# EUROPEAN PATENT APPLICATION

(11) **EP 4 428 869 A1**
(43) Date of publication of application: **11.09.2024**
(21) Application number: 23160357.2
(22) Date of filing: 07.03.2023
(51) Int. Cl.: G16H 20/10, G16H 50/20, A61K 31/56, A61K 31/4375, C07D 215/44, C07C 251/58, C07D 307/80, C07D 413/04

(54) **METHODS FOR DETERMINING DRUG CANDIDATES**

(71) Applicant: mosaiques diagnostics & therapeutics AG, 30659 Hannover (DE)
(72) Inventor: Mokou, Marika, 30659 Hannover (DE); Mischak, Harald, 30659 Hannover (DE)
(74) Representative: Kröncke, Rolf

(57) **Abstract**

The present invention relates in a first aspect to a method for determining candidate molecules for treating a disease, disorder or condition associated with tissue alteration. In particular, the method refers to determining candidate molecules for treating bladder cancer in different stages of the disease, including an *in silico* prediction of drug response based on reversion of a disease specific molecular signature. For example, the CMap analysis is applied on data sets obtained based on proteomics and transcriptomics analyses.

In a further aspect, the present invention relates to candidate molecules useful for treating said disease, disorder or condition at different stages of disease, in particular, allowing reversal of a more severe stage of disease to a more moderate stage or to stop progression to aggressive phenotypes like aggressive phenotype of bladder cancer. Finally, drug candidates useful in the treatment of said disease, in particular, the treatment of bladder cancer are provided.

## Description

The present invention relates in a first aspect to a method for determining candidate molecules for treating a disease, disorder or condition associated with tissue alteration. In particular, the method refers to determining candidate molecules for treating bladder cancer in different stages of the disease, including an *in silico* prediction of drug response based on reversion of a disease specific molecular signature. For example, the CMap analysis is applied on data sets obtained based on proteomics and transcriptomics.

In a further aspect, the present invention relates to candidate molecules useful for treating said disease, disorder or condition at different stages of disease, in particular, allowing reversal of a more severe stage of disease to a more moderate stage or to stop progression to aggressive phenotypes like aggressive phenotype of bladder cancer. Finally, drug candidates useful in the treatment of said disease, in particular, the treatment of bladder cancer are provided.

### Prior Art

Drug repurposing aims at the application of existing drugs for different purposes from their original context of use. This is a cost-effective strategy towards new therapies accelerating clinical translation in new applications. Namely, drug repurposing represents a cost-effective and efficient manner for new therapies against diseases, disorders and conditions not yet treated with said drugs. Due to the fact that the drugs have been already assessed for their safety and tolerability, drug repurposing shortens the drug development timelines, increases the odds of success, expedites regulatory approval and reduces the overall costs from bench to bedside. Namely, 30 % of all drugs approved by the US Food and Drug Administration represent repurposed drugs. Strategies for repurposing, can be established by combining machine-learning compilation methods with chemical structures, genotypes, large-scale transcriptomics, proteomics data, e. g. Pushpakom S., et al., Nat.Rev.Drug.Discov., 2019, 18 (1): 41-58. Several resources have been introduced in the recent years for data-driven drug repurposing with the Connectivity Map (CMap) being one of the most well-known tools for signature matching drug repurposing. CMap was released in 2006 and is freely available via https://clue.io/. Through a pattern matching algorithm CMap establishes similarities among drugs based on gene expression profiles observed in disease affected cell lines, like cancer cell lines. Specifically, CMap relies on the signature reversion principle and allows researchers to interrogate their own gene list of interest, also called signature, against a large reference gene expression database that was generated by perturbing cell lines with a range of chemical compounds to identify drugs and new expression patterns similar (or opposite) to that of a query list. CMap analysis is typically based on data at the transcriptome level which normally constitutes a limitation of the approach when using as input other -omics, like proteomics, data. CMap has been applied for the identification of repurposed drugs in a wide range of diseases, based on single omics data.

Bladder cancer (BC) remains one of the most prevalent cancers globally, despite the significant progress that has been made in diagnosis, treatment and care in the recent years. BC is one of the diseases with highest lifetime treatment costs per patient. Typically, BC is histologically classified into Non-Muscle Invasive BC (NMIBC) and Muscle Invasive BC (MIBC). NMIBC accounts for approximately 75 % of the newly diagnosed BC cases. Based on clinical pathological characteristics stratification of NMIBC to subgroups of low, intermediate and high risk of progression is applied. The standard of care for patients with low risk NMIBC is transurethral resection of the bladder tumor (TURBT) whereas for intermediate and high risk tumors TURBT should be followed by intravesical bacillus Calmette-Guérin (BCG) immunotherapy or instillations of chemotherapy for one to three years to reduce the frequency of recurrence and progression to MIBC. Nevertheless, almost 40 % of the NMIBC patients fail to respond to first line BC therapy. The latter along with the high-recurrence rate of non-muscle invasive tumors (50 % to 70 %) and their progression to muscle invasive disease represent big challenges of current therapeutic approaches. Of the early diagnosed patients with BC, 25% will present with MIBC or metastatic disease. The gold standard of treatment for patients with muscle invasive non-metastatic BC remains cisplatin-based neoadjuvant (NAC) chemotherapy followed by radical cystectomy with lymph node dissection. Further, efficacy of immune checkpoint inhibitors (ICI) was recently reported and transformed the treatment landscape of advanced BC. Nevertheless, a high proportion of patients show low response rates and/or experience adverse events, which further necessitates improved patient stratification methods and better management of the observed ICI-related toxicity. Bladder disease management for MIBC as well as high risk NMIBC in cost-effective manners is urgently needed. In view of the high recurrence and the challenges of the current therapeutic schemes as well as acquisition of resistance and presence of numerous side-effects, it becomes evident that there is an urgent need for the identification of novel effective therapeutic strategies especially at earlier phases of BC, ideally before tumor has progressed to MIBC.

Recently CMap has been applied in the context of BC where a CMap based drug repurposing pipeline based on patients omic signature has been described, Mokou, M., et al, Cancers (Basel). 2020 Nov 26;12(12):3519. However, the described pipeline was restricted to tissue proteomics data from patients with high-risk and low-risk Non-Muscle Invasive BC (NMIBC) but missed including information on the specific changes observed in tumor cells only (of note: the tumor cells only are the target for intervention), e.g. by including omics data from *in vitro* studies.

### Description of the present invention

The present inventors recognized that differences in stages and aggressiveness of a disease, disorder or condition can be characterized based on different -omic signatures using tissue proteomics, transcriptomics and omics data retrieved from various sources including tissue but also applying proteomics and transcriptomics data from BC cell lines allowing to determine candidate molecules, in particular, allowing repurposing of drugs for treating said disease, disorder or condition. By prediction of the drug response based on reversion of a disease specific molecular signature it is possible to determine suitable candidate molecules, in particular, allowing repurposing of the drugs accordingly. In particular, by combining the different signatures including the proteomics and transcriptomics data from cell lines, problems accruing in the past can be addressed. Namely, analysing tissue only whereby said tissue typically comprises multiple cell types on not only disease, disorder or conditions of specific cells, the data obtained may be falsified. This is particularly true when not only analysing the disease affecting cell or cell lines but even non cellular material present in the tissue is analysed including blood, plasma and extracellular matrix contributing to the final result. The present invention comprises the comparison of tissue data with data from specific cell lines restricting the information specifically obtained from the diseased cell. Particularly, in case of cancer cells and e.g. BC cells, the approach according to the present invention allows detecting credible candidate molecules to be tested as BC drugs accordingly. The molecular BC signatures restricted to features that are also present in BC cell lines were investigated in the examples through a drug repurposing pipeline employing the CMap tool resulting in the identification of candidate molecules with potential to reverse the disease signature.

That is, the present invention relates to a first aspect to a method for determining candidate molecules for treating a disease, disorder or condition associated with tissue alteration, like a tumor, in particular bladder cancer (BC) at different stages of said disease, disorder or condition comprising in silico prediction of drug response, based on reversion of a disease specific molecular signature, in particular, applying CMap analysis based on data obtained by
i) a first method of obtaining a disease, disorder or condition specific signature, like BC signatures from the altered tissue, like from BC tissue whereby a) these tissues are analyzed on proteomic differences associated with disease, disorder or condition associated progression, like tumor progression, in particular with BC progression and be detected in more than 10 % of the analyzed tissue samples followed by b) identifying features characterizing different protein expression associated with the progression or the different stages of the disease, disorder or condition for providing a first data set for *in silico* prediction of drug response, in particular by CMap analysis;
ii) a second method of obtaining a disease, disorder or condition specific signature, like BC signatures whereby a) the cell proteome of cell lines, like tumor cell lines, in particular, BC cell lines depicting MIBC and NMIBC pathology, of different stages of progression of said disease, disorder or condition are analyzed and determining proteins demonstrating significant change in abundance between different stages of progression followed by b) comparing the protein of the cells, like the tumor cells, in particular, the BC, with the tissue proteome for providing a second data set for in silico prediction of drug response, in particular by CMap analysis;
iii) a third method of obtaining a third disease, disorder or condition specific signature, like a third BC signature comprising a) obtaining a cell lines transcriptomics data set whereby cell lines of different stages of the disease, disorder or condition are analysed; b) obtaining a cell line proteomics data set whereby cell lines of different stages of the disease, disorder or condition are analysed; c) comparing the proteomics data and transcriptomic data of a) and b) for obtaining features common in both data sets; and d) from the common features obtained in c) determining features being statistically significant different in at least one of the data set of a) and b)for providing a third data set for in silico prediction of drug response, in particular by CMap analysis; and
iv) the data set obtained in each of i), ii) and iii) are analyzed by a method for *in silico* prediction of drug response, in particular by CMap analysis for determining candidate molecules.

In this connection, the term "candidate molecules for treating a disease, disorder or condition associated with tissue alteration" refers to molecules or compounds that may have an effect on the disease, disorder or condition. For example, in case of tumors in particular, in case of BC, said candidate molecules represent compounds or molecules which demonstrate an effect on parameters associated with said tumor, like BC. In particular, according to the present invention it is possible that these candidate molecules display the activity on reversing more severe stages of the disease to less severe stages or do stop progression to aggressive phenotypes like aggressive phenotype of BC.

The terms compounds and molecules are used herein interchangeably unless otherwise indicated.

The term "reversion of a disease specific molecular signature" refers to the potential of reversing a specific expression profile of a given set of hallmark genes or proteins for a particular disease. The reversion includes embodiments with reversion from different stages in a disease, disorder or condition as well as reversion from a severe to a less severe situation within a single stage of the disease, disorder or condition. For example, in BC reversion may be from MIBC to NMIBC or within NMIBC from the severe subgroup to a moderate or low subgroup.

According to step i) of the method according to the present invention, tissues are analyzed to identify proteomic differences associated with the disease, disorder or condition and/or associated with progression of the same. For example, the progression includes the tumor progression or, for example in case of BC the progression from non-muscle-invasive bladder cancer (NMIBC) to muscle-invasive bladder cancer (MIBC), or in between the different stages of the NMIBC as identified above.

By detecting said proteomic differences resulting in identifying a number of proteins is present in more than 10 % of the analyzed tissue samples, it is submitted that this is a trend of expression of the protein rather than a single event in one tissue sample. The first method according to step i) continued by identifying features characterizing different protein expression associated with a progression of the disease, disorder or condition. These features represent a first data set for the *in silico* prediction of drug response, like CMap analysis.

As used herein, the term "features" refers to molecular markers including RNA or proteins that further determine the characteristics of cells or tissues. Typically, the features derived from transcriptomic data are RNA or modification of nucleic acids, in particular, RNA while features of proteomics are peptides and proteins as well as modifications thereof.

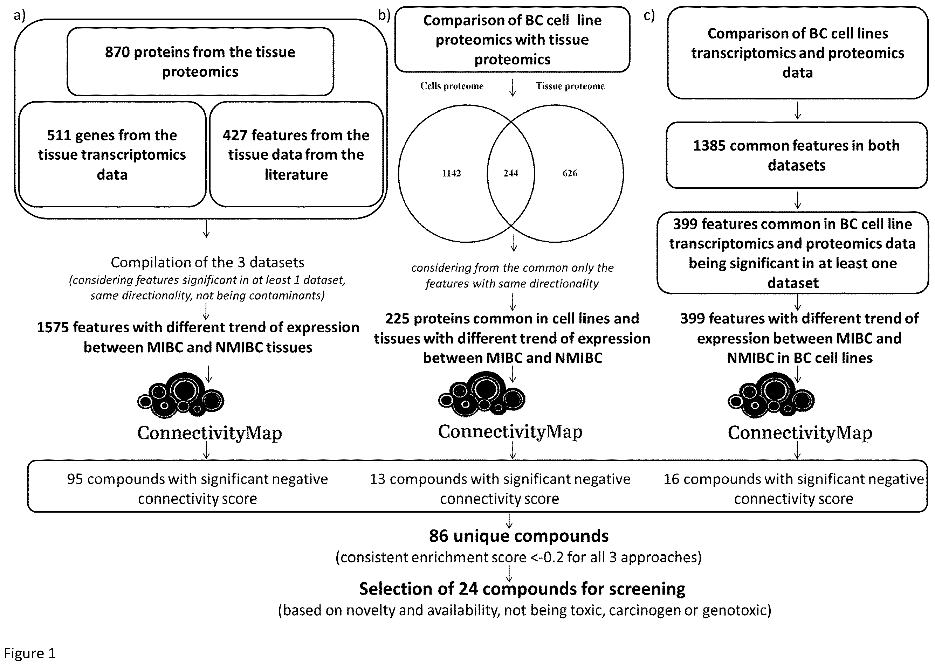

Further, the first method may be exemplified to identify drug candidates having the potential to reverse the aggressive phenotype of BC and thus can be repurposed for BC treatment on the basis of different -omics signatures using tissue proteomics, transcriptomics and additional data (e.g. from data repositories, from literature, etc. which are publically available) and analyzing them *in silico.* The different data may be obtained from public sources including tissue data as well as proteomics and transcriptomics data from cell lines like BC cell lines. For example, the first method of step i) may be conducted as depicted in Figure 1 as follows:
Raw proteomic data of 117 BC tissues were received from public sources Stroggilos, R., et al., Int. J. Cancer 2020, 146(1): 281 - 294 and were further processed and evaluated focusing on the proteomic differences associated with BC progression, namely, differences between MIBC and NMIBC. The data were analyzed with three different analytical approaches [(Proteome Discoverer 1 % FDR and no FDR threshold) and MaxQuant (1 % FDR) given the complementarity of these approaches, generated data were compiled, while several stringency criteria were considered for shortlisting the differently abundant proteins including adjusted P-value using the Benjamini-Hochberg-method to adjust for multiple testing, frequency and fold change agreement among different analyses. As identified in step i) of the method according to the present invention, these proteins were detected in > 10% of the samples with agreement in their trend of expression/abundance among the three different analytical approaches described above. These proteins were subsequently integrated with tissue transcriptomics and literature mined data from public sources, e.g. public databases or from literature; e.g. a search in the literature for previously published studies describing differences between MIBC and NMIBC at the RNA and protein level was done. Then all the data from the literature were integrated and this is defined as "literature mined data". Further, the tissue transcriptomics data derived from the successful compilation of available transcriptomics/ gene expression datasets from different repositories (cBioportal, GEO, ArrayExpress) Stroggilos R, et al. Cancers (Basel). 2022. 14(10):2542. As such, gene expression profiles from 1,054 primary BC tumor transcriptomes of treatment-naive patients without any prior cancer history, along with profiles from 81 non-malignant urothelium tissues adjacent to the tumor site (NAU), generated using Affymetrix and Illumina platforms, were extracted. Data were processed, normalized, followed by the removal of batch effect using ComBat method and statistical analyses. Differentially expressed genes related to BC progression (MIBC vs NMIBC) were defined. Thus, the analysis and compilation of the data result in a multitude of features, in the present case 1575 features with different trend of expression between MIBC and NMIBC comprising the molecular signature of BC. This molecular signature of BC represents the first data set for the *in silico* prediction of drug response, in particular by CMap analysis.

According to step ii) the second method, see e.g. Fig. 1b), is applied whereby cell lines like tumor cell lines, in particular, BC cell lines, are analyzed depicting differences in progression, like depicting different stages of tumor progression with the goal of determining proteins demonstrating significant changes in expression between the different stages of progression, for example, with BC, the different stages are MIBC and NMIBC. The obtained proteins with difference in progression were compared with the tissue proteome.

The comparison of the tissue with the cell proteome reveals proteins having the same trend of expression when comparing MIBC with NMIBC accordingly. This additional signature of BC is used as the second data set for in silico prediction of drug response, in particular, by CMap analysis.

In step iii) of the method according to the present invention, see Fig. 1c), a third method is applied. Namely, this third method provides a third signature which is a data set allowing further *in silico* prediction of drug response, in particular, by CMap analysis. In this third method proteomics data and transcriptomics data of cell lines associated with the disease, disorder or condition are combined for obtaining features being statistically different comparing different progression states of the disease, disorder or condition for each data set of transcriptomics and proteomics data.

Namely, the cell lines transcriptomics data set is obtained whereby cell lines of different stages of the disease, disorder or condition are analysed. Further the cell line proteomics data set are obtained whereby cell lines of different stages of the disease, disorder or condition are analysed. Moreover, by comparing the proteomics data and transcriptomic data above, features common in both data sets are determined. Thereafter, among the common features obtained above, those being statistically significant different in at least one of the above transcriptomics and proteomics data set are determined providing a third data set for in silico prediction of drug response, in particular by CMap analysis.

The data sets obtained by each of the methods of step i), ii) and iii) are analyzed further in step iv) by a method of *in silico* prediction of drug response, in particular, by CMap analysis allowing to determine candidate molecules accordingly. In the example shown in Figure 1, the analysis resulted in identifying a number of compounds with negative connectivity score. The identified compounds were further analysed on various aspects including toxicity, etc., and specific compounds were determined.

In an aspect, these candidate molecules are repurposed drugs.

In an embodiment of the present invention, the disease, disorder or condition is a tumor, like solid tumor. In an embodiment, the solid tumor is particularly BC. BC progression is differentiated between NMIBC and MIBC.

In an embodiment, candidate molecules are molecules being determined on their ability to allow reversal of an aggressive phenotype of the disease, disorder or condition associated with tissue alteration, like a tumor, in particular, BC to a less aggressive phenotype. For example, in case of BC the candidate molecule is considered to be able to inhibit the conservation to the MIBC scenario and reverse BC to less severe stage.

In an embodiment of the present invention, in step i) the proteins obtained from tissue proteomics are compared with the differently expressed genes relevant to stage progression from tissue transcriptomics. For example, the proteins obtained from the tissue proteomics showing differences in the progression of the disease are compared to tissue transcriptomics representing different stages of progression of said disease accordingly. For example, in case of BC the stages include NMIBC and MIBC accordingly.

In an embodiment of the present invention, the cell lines analyzed in the second method as identified in step ii) are cell lines having either NMIBC or MIBC phenotypes. The cell lines that were analyzed included the HBLAK and RT112 cells which were both annotated as derived from patients with NMIBC as well as the BFTC-905, HT1376 and T24 cells that were all annotated as derived from patients with MIBC.

Moreover, in an embodiment of the present invention the method is characterized in that proteins determined in step ii) include among others proteins for which disease specificity in the analysed tissue, like tumor association is known.

Further, according to an embodiment of the present invention, the tissue proteome used in the method according to step ii) is the tissue proteome of step i). That is, an identical tissue proteome is used.

In another embodiment, the transcriptomics used in step iii) is a transcriptomics from cell lines from urinary tract when looking for candidate molecules of treating BC.

In an embodiment, the *in silico* step of predicting drug response based on the data sets obtained in each of the steps i), ii) and iii) is by CMap analysis. For example, the candidate molecules are determined by a negative enrichment score during CMap analysis. The skilled person is well aware of methods to identify the same and determining negative enrichment scores accordingly.

In an embodiment, the method includes furthermore the step of determining data of treating a disease, disorder or condition, associated with tissue alteration, like a tumor, in particular, BC of the candidate molecules identified. in particular, data present in database or otherwise publicly available of the candidate molecules in treating a different disease, like in treating cancer, in particular, cancer other than BC or known candidate molecules in treating different (other than cancer) diseases.

The method according to the present invention allows identifying suitable candidate molecules for further testing of effectiveness and efficacy in treating the respective disease, disorder or condition. In particular, repurposing of known drugs described for treating different diseases, disorders or conditions, in particular, non-related diseases, disorders or conditions, are identified and determined according to the present invention.

In a further aspect, the present invention relates to the candidate molecule obtainable by a method according to the present invention.

For example, the candidate molecule according to the present invention being a compound of general formula I 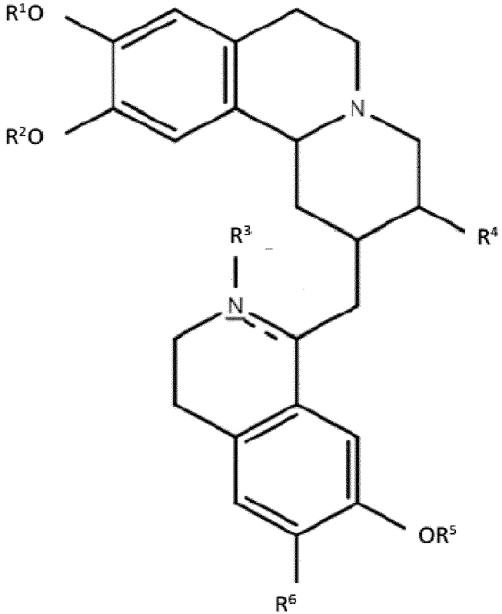 wherein
R1, R2, R5 are independently selected from H, C1-C9 alkyl, e.g. C1 - C6 alkyl, like C1-C4 alkyl;
R4, R6 is selected from H, C1-C9 alkyl, e.g. C1 -C6 alkyl, like C1-C4 alkyl, OC1-C9 alkyl, e.g. OC1 - C6 alkyl, like OC1-C4 alkyl;
R3 is absent and ----- is a double bond, or R3 is present when ----- is a single bond with R3 being selected from H, C1-C9 alkyl, e.g. C1-C6 alkyl, like C1-C4 alkyl,
for use in the treatment of BC.

For example, the candidate molecule is cephaeline, emetine, psychotrine, o-methyl psychotrine or emetamine.

In another embodiment, the drug candidate is a compound of general formula II 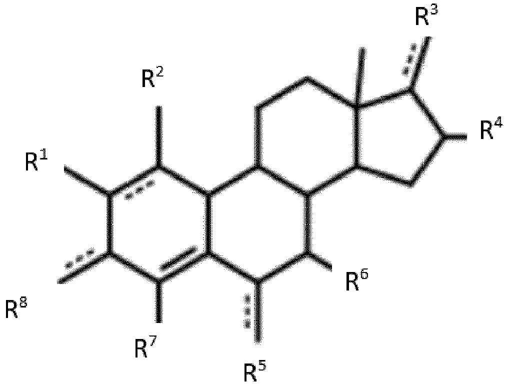 wherein
R1, R2, R4, R6 and R7 each are independently selected from H, C1 - C4 alkyl, substituted C1 - C4 alkyl, halogen;
R3 and R8 are each individually selected from O, OH, O C1 - C4 alkyl;
----- is a single or double bond;
R5 is a compound of CHn(C1 - C4)m with n and m being an integer of 0 to 3 and n + m is 3
when ----- is a single bond, and
R5 is CH2, CH C1 - C4 alkyl or C (C1 - C4 alkyl)2 when ----- is a double bond,
for use in the treatment of BC.

In particular, the compound of formula II is exemestane.

In addition, according to the present invention, other favorable candidate molecules include compounds from the group of fluvoxamine or a derivative thereof; amiodarone or a derivative thereof, isradipine or a derivative thereof, or amodiaquine or a derivative thereof.

In a preferrred embodiment, the candidate molecule in the treatment of BC is cephaeline, in another embodiment, the candidate molecule is exemestane.

The term "derivative thereof" refers to compounds which can be synthesized by chemical transformations of the compounds functional groups using standard reactions.

In addition, unless otherwise indicated, the candidate compounds may be present in the form of a prodrug, metabolite or derivative. Further, the candidate compounds may be present in the form of pharmaceutically acceptable salts or hydrates.

Based on the candidate molecules determined in the method of the present invention further selection of possible candidate molecules is possible. For example, the candidate molecules are searched for known information on biological effectiveness and, in particular, any disease association. This allows focusing on suitable compounds as candidate molecules for drugs.

The candidate molecules or drug candidates are screened for their efficacy on BC cells or tumors. For example, in the present case, a list of twenty four compounds was identified as given below.

**Table 1. The list of the 24 compounds which were shortlisted for in vitro screening.**

| **Name of the compound** | **Class** |
|---|---|
| **ursodeoxycholic acid (synonym: ursodiol)** | a secondary bile acid |
| **cephaeline** | alkaloid with emetic properties |
| **sulfamethoxypyridazine** | a sulfonamide antibacterial |
| **guanethidine** | antihypertensive |
| **exemestane** | aromatase inhibitor member of the class of antiestrogens |
| **DL-thiorphan** | enkephalinase inhibitor |
| **1,4-chrysenequinone** | activator of aryl hydrocarbon receptor (AhR) |
| **fenspiride** | Antiasthmatic & COPD Preparations |
| **morantel** | anthelmintic drug |
| **latamoxef** | Antibacterial agents |
| **flavoxate** | anticholinergic with antimuscarinic effects |
| **propantheline bromide** | anticholinergic with antimuscarinic effects |
| **tubocurarine chloride** | neuromuscular blocking agents |
| **metolazone** | diuretics |
| **sulfanilamide** | anti-infective |
| **demeclocycline** | antibiotic |
| **methylergometrine** | ergot alkaloids |
| **bupropion** | antidepressants |
| **fluvoxamine** | selective serotonin reuptake inhibitors (SSRIs) |
| **amiodarone** | antiarrhythmics |
| **primidone** | anticonvulsants |
| **iobenguane** | Antineoplastic Agents - Radiopharmaceutical |
| **amodiaquine** | antimalarial drug |
| **isradipine** | Antihypertensive calcium channel blockers |

These twenty four compounds were selected based on the list of candidate molecules. Some of these drug candidates have been previously used in other cancer types, further supporting the potential to provide a positive result during the studies in BC. The compounds shown in Table 1 belong to different drug classes as outlined.

The impact of the drug candidates onto the malignant phenotype of BC cells was assessed *in vitro* in a panel of multi-origin BC cell lines representing different stages during disease progression. These included non-muscle invasive (RT112) and muscle invasive (T24, BFTC905, HT1376) cells.

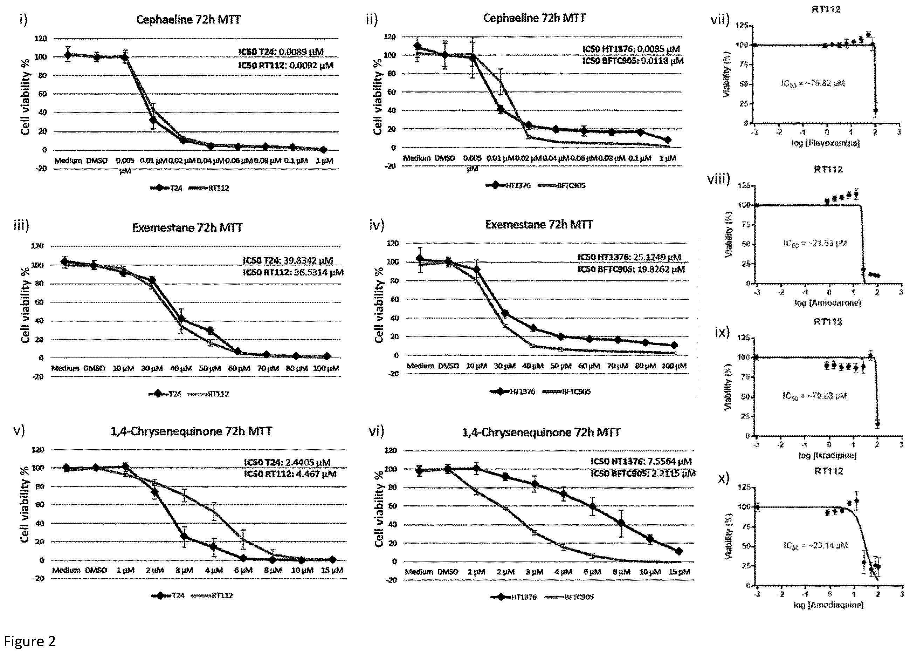

Of the twenty four compounds, cephaeline, exemestane, 1,4- chrysenequinone, amodiaquine, isradipine, amiodarone and fluvoxamine prevented cell growth at relatively low concentrations. Accordingly, these compounds were selected for further investigation, here providing the response curves for four different BC cell lines. Representative response curves of the seven compounds are presented in Figure 2, along with the respective IC50s. A clear impact was observed on the proliferation of the BC cells for all seven compounds.

That is, the seven drugs significantly decreased the growth rate of the BC cells *in vitro.*

In the next step, the impact of the seven compounds is investigated *in vivo* in xenograft mouse models.

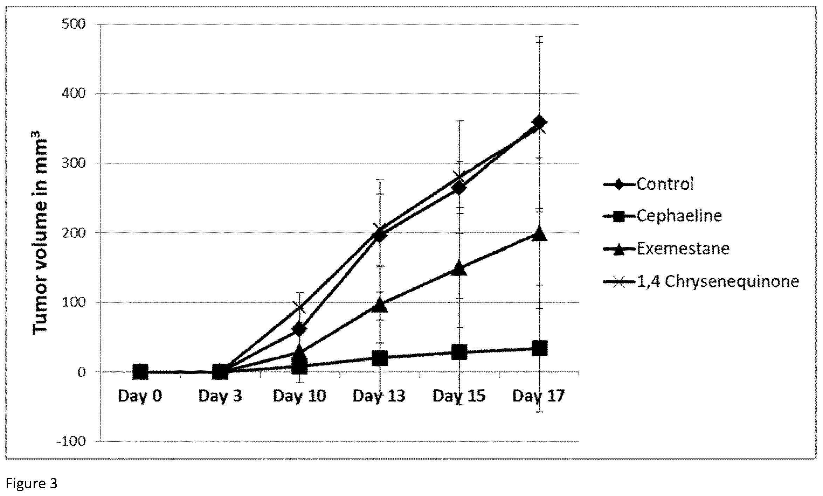

In vivo testing of exemestane, cephaeline and 1,4 chrysenequinone was performed in xenograft mouse models. Particularly, the model was established after injecting 4*10⁶ BFTC905 cells subcutaneously into the tail base of NOD/SCID male mice. Male mice were selected for these experiments since males are more prone to develop BC. The treatments started three days after the injections of the cancer cells. The treatment scheme was 30mg/kg/every 3rd day for exemestane, 5mg/kg/daily for cephaeline and 5mg/kg/daily for 1,4 chrysenequinone. As controls were used animals injected with DMSO diluted in PBS (at a final concentration similar to the one of the compounds). As shown in Figure 3, a clear impact on tumor establishment or growth was observed for cephaeline and exemestane respectively. The tumor volume of the animals treated with 1,4 chrysenequinone was similar to the one of the control-DMSO group, indicating lack of efficacy of this drug *in vivo.* The impact of the drugs was observed at least for 17 days until the tumor volume of the control animals reached >300mm³.

Based on the above, the present inventors identified cephaeline, exemestane as suitable drug candidates or candidate molecules for treating BC. In particular, it is submitted that these compounds as well as derivatives thereof are suitable for reversal of an aggressive phenotype of BC to a less aggressive phenotype for inhibiting progression to a more severe phenotype accordingly.

Moreover, by applying the method according to the present invention, the inventors recognized further compounds representing suitable candidate molecules. Said compounds are selected from the group of
i) Fluvoxamine or a derivative thereof;
ii) Amiodarone or a derivative thereof;
iii) Isradipine or a derivative thereof; or
iv) Amodiaquine or a derivative thereof.

### Brief description of the figures:

**Figure 1****.** Schematic representation of the workflow followed for the generation of BC signatures that were then used for the identification of drugs that have the potential to reverse the aggressiveness of BC. a) Combination of tissue proteomics, transcriptomics and tissue data (RNA and protein expression data) retrieved from the literature; b) Combination of BC cell lines proteome with tissue proteome; c) Compilation of BC cell lines transcriptomics and proteomics data. The three different signatures were used as input for CMap analyses. Using a consistent enrichment score <-0.2 for all 3 approaches was applied. This resulted in 86 candidates being shortlisted. All candidates were investigated. Sixty-two of them were not further considered based on a) already described in the context of bladder cancer b) not available to be purchased or c) being toxic, carcinogen or genotoxic. The remaining 24 compounds were further investigated in *in vitro* systems (bladder cancer cell lines).
**Figure 2****.** The dose response curves of cephaeline (i-ii), exemestane (iii-iv) and 1,4 chrysenequinone (v-vi) in the BC cell lines RT112, T24, HT1376 and BFTC905. The dose response curves of fluvoxamine (vii), amiodarone (viii), isradipine (ix) and amodiaquine (x) in the RT112 cells are also provided. All of the drugs significantly decreased the proliferation rate of the BC cells *in vitro.*
**Figure 3****.** The impact of cephaeline, exemestane and 1,4 chrysenequinone *in vivo* in BFTC905 xenograft mouse models. Cephaeline and exemestane significantly decreased tumor establishment or growth *in vivo* whereas no significant impact on tumour growth was observed for 1,4 chrysenequinone.

## Claims

1. A method for determining candidate molecules for treating a disease, disorder or condition associated with tissue alteration, like a tumor, in particular bladder cancer (BC) at different stages of said disease, disorder or condition comprising in silico prediction of drug response, based on reversion of a disease specific molecular signature, in particular, applying CMap analysis based on data obtained by
i) a first method of obtaining a disease, disorder or condition specific signature, like BC signatures from the altered tissue, like from BC tissue whereby a) these tissues are analyzed on proteomic differences associated with disease, disorder or condition associated progression, like tumor progression, in particular with BC progression and be detected in more than 10 % of the analyzed tissue samples followed by b) identifying features characterizing different protein expression associated with the progression or the different stages of the disease, disorder or condition for providing a first data set for in silico prediction of drug response, in particular by CMap analysis;
ii) a second method of obtaining a disease, disorder or condition specific signature, like BC signatures whereby a) the cell proteome of cell lines, like tumor cell lines, in particular, BC cell lines depicting MIBC and NMIBC pathology, of different stages of progression of said disease, disorder or condition are analyzed and determining proteins demonstrating significant change in abundance between different stages of progression followed by b) comparing the protein of the cells, like the tumor cells, in particular the BC, with the tissue proteome for providing a second data set for in silico prediction of drug response, in particular by CMap analysis;
iii) a third method of obtaining a third disease, disorder or condition specific signature, like a third BC signature comprising a) obtaining a cell lines transcriptomics data set whereby cell lines of different stages of the disease, disorder or condition are analysed; b) obtaining a cell line proteomics data set whereby cell lines of different stages of the disease, disorder or condition are analysed; c) comparing the proteomics data and transcriptomic data of a) and b) for obtaining features common in both data sets; and d) from the common features obtained in c) determining features being statistically significant different in at least one of the data set of a) and b) for providing a third data set for in silico prediction of drug response, in particular by CMap analysis; and
iv) the data set obtained in each of i), ii) and iii) are analyzed by a method for in silico prediction of drug response, in particular by CMap analysis for determining candidate molecules.

2. The method for determining candidate molecules for treating a disease, disorder or condition, associated with tissue alteration, like a tumor, in particular BC according to claim 1 wherein BC progression is differentiated between non-muscle invasive bladder cancer (NMIBC) and muscle invasive bladder cancer (MIBC).

3. The method for determining candidate molecules for treating a disease, disorder or condition, associated with tissue alteration, like a tumor, in particular BC according to claim 1 or 2 wherein the candidate molecules are determined to allow reversal of an aggressive phenotype of the disease, disorder or condition, associated with tissue alteration, like a tumor, in particular BC.

4. The method for determining candidate molecules for treating a disease, disorder or condition, associated with tissue alteration, like a tumor, in particular BC according to any one of the preceding claims wherein in step i) after b) the features of b) are compared with tissue transcriptomics data and/or features from tissue databases for providing features differentially expressed at the different stages of said disease, disorder or condition, like being relevant to stage progression, in particular, BC progression present in the first data set.

5. The method for determining candidate molecules for treating a disease, disorder or condition, associated with tissue alteration, like a tumor, in particular BC according to any one of the preceding claims wherein the cell lines analyzed in method ii) are cell lines having either MIBC or NMIBC phenotype.

6. The method for determining candidate molecules for treating a disease, disorder or condition, associated with tissue alteration, like a tumor, in particular BC according to any of the preceding claims wherein proteins determined in step ii) included among others proteins for which disease specificity in the analysed tissue, like tumor association, is known.

7. The method for determining candidate molecules for treating a disease, disorder or condition, associated with tissue alteration, like a tumor, in particular BC according to any one of the preceding claims wherein the tissue proteome used in step ii) is the tissue proteome of step i).

8. The method for determining candidate molecules for treating BC according to any one of the preceding claims wherein the transcriptome in step iii) is RNA transcriptome from cell lines from urinary tract.

9. The method for determining candidate molecules for treating a disease, disorder or condition, associated with tissue alteration, like a tumor, in particular BC according to any one of the preceding claims wherein in step iv) candidate molecules are determined by a negative enrichment score in CMap analysis.

10. The method for determining candidate molecules for treating a disease, disorder or condition, associated with tissue alteration, like a tumor, in particular BC according to any one of the preceding claims further comprising the step of determining data on treating a disease, disorder or condition, associated with tissue alteration, like a tumor, in particular BC of said candidate molecules, in particular with known candidate molecules in treating cancer, in particular, cancer other than BC or known candidate molecules in treating different (other than cancer) diseases.

11. A candidate molecule obtained by a method according to any one of claims 1 to 10.

12. The candidate molecule according to claim 11 being a compound of general formula I 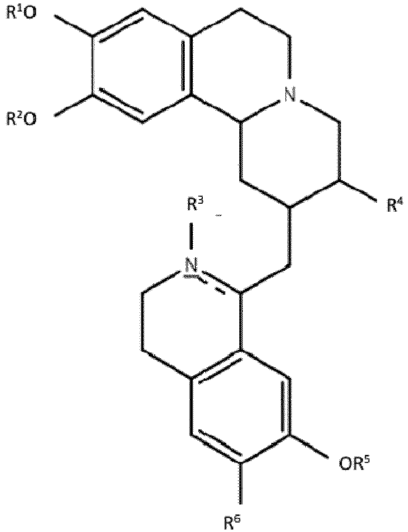 wherein
R1, R2, R5 are independently selected from H, C1-C9 alkyl, e.g. C1 - C6 alkyl, like C1-C4 alkyl;
R4, R6 is selected from H, C1-C9 alkyl, e.g. C1 - C6 alkyl, like C1-C4 alkyl, OC1-C9 alkyl, e.g. OC1 - C6 alkyl, like OC1-C4 alkyl;
R3 is absent and ----- is a double bond, or R3 is present when ----- is a single bond with R3 being selected from H, C1-C9 alkyl, e.g. C1-C6 alkyl, like C1-C4 alkyl,
for use in the treatment of BC.

13. The candidate molecule according to claim 12 for use in the treatment of BC being Cephaeline.

14. The drug candidate according to claim 11 being a compound of general formula II 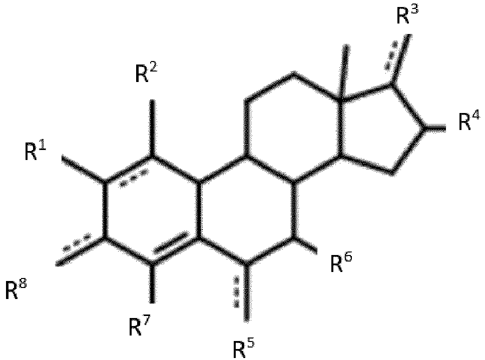 wherein
R₁, R₂, R₄, R₆ and R₇ each are independently selected from H, C₁ - C₄ alkyl, substituted C₁ - C₄ alkyl, halogen;
R₃ and R₈ are each individually selected from O, OH, O C₁ - C₄ alkyl;
----- is a single or double bond;
R5 is a compound of CHn(C1 - C4)m with n and m being an integer of 0 to 3 and n + m is 3 when ----- is a single bond, and
R5 is CH2, CH C1 - C4 alkyl or C (C1 - C4 alkyl)2 when ----- is a double bond, for use in the treatment of BC.

15. The candidate molecule of general formula II for use in the treatment of BC according to claim 14 wherein R₁, R₂, R₄, R₆ and R₇ are hydrogen; R₃ and R₈ are O and R₅ is CH₂ ----- is a double bond, in particular, is exemestane.

16. The candidate molecule according to claim 11 being a compound selected from the group of
v) Fluvoxamine or a derivative thereof;
vi) Amiodarone or a derivative thereof;
vii) Isradipine or a derivative thereof; or
viii) Amodiaquine or a derivative thereof.
